(19) Europäisches Patentamt
European Patent Office
Office européen des brevets

(11) **EP 4 201 523 A1**

(12) **EUROPÄISCHE PATENTANMELDUNG**

(43) Veröffentlichungstag:
28.06.2023  Patentblatt 2023/26

(21) Anmeldenummer: 21216649.0

(22) Anmeldetag: **21.12.2021**

(51) Internationale Patentklassifikation (IPC):
**B01J 35/00** (2006.01)   **B01J 23/44** (2006.01)
**B01J 23/52** (2006.01)   **C07C 67/055** (2006.01)

(52) Gemeinsame Patentklassifikation (CPC):
(C-Sets verfügbar)
**B01J 35/1038; B01J 23/44; B01J 23/52;
B01J 35/008; B01J 35/1019; B01J 37/0205;
B01J 37/0223; B01J 37/024; C07C 67/055**  (Forts.)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Clariant International Ltd**
**4132 Muttenz (CH)**

(72) Erfinder:
• **BOBKA, Roman**
  **83026 Rosenheim (DE)**
• **MESTL, Gerhard**
  **80935 Muenchen (DE)**
• **SCHECK, Peter**
  **82205 Gilching (DE)**

(74) Vertreter: **Klingelhöfer, Stefan**
**Clariant Produkte (Deutschland) GmbH
IPM / Patent & License Management
Arabellastraße 4a
81925 München (DE)**

(54) **SCHALENKATALYSATOR ZUR HERSTELLUNG VON ALKENYLCARBONSÄUREESTERN MIT VERBESSERTER PD-VERTEILUNG**

(57)    Die vorliegende Erfindung betrifft einen Pd- und Au-haltigen Schalenkatalysator, der sich durch eine verbesserte Verteilung des Pd auszeichnet. Außerdem betrifft die Erfindung auch zwei Verfahren zur Herstellung dieses Katalysators sowie ein Verfahren zur Herstellung von Vinylacetat-Monomer unter Verwendung dieses Katalysators.

Fig. 1

**(Forts. nächste Seite)**

EP 4 201 523 A1

(52) Gemeinsame Patentklassifikation (CPC): (Forts.)

C-Sets
**C07C 67/055, C07C 69/15**

**Beschreibung**

Die vorliegende Erfindung betrifft einen Pd- und Au-haltigen Schalenkatalysator, der sich durch eine verbesserte Verteilung des Pd auszeichnet. Außerdem betrifft die Erfindung auch zwei Verfahren zur Herstellung dieses Katalysators sowie ein Verfahren zur Herstellung von Vinylacetat-Monomer unter Verwendung dieses Katalysators.

Schalenkatalysatoren finden seit Jahren Anwendung in verschiedenen katalytischen Verfahren. Sie zeichnen sich dadurch aus, dass die katalytisch aktiven Spezies nicht über den gesamten Katalysatorträger verteilt sind, sondern lediglich in einem schalenförmigen Bereich um den Mittelpunkt des Katalysatorträgers.

Ein Anwendungsgebiet betrifft die Herstellung von Vinylacetatmonomer (VAM) durch die Reaktion von Ethylen, Essigsäure und Sauerstoff. Hierbei werden verbreitet Schalenkatalysatoren eingesetzt, die als katalytisch aktive Spezies Pd- und Au-Verbindungen enthalten und darüber hinaus noch verschiedene Promotoren enthalten können.

So beschreibt die WO 2008/145388 A1 die Herstellung von Pd- und Au-haltigen Schalenkatalysatoren, bei denen die Aufbringung der katalytisch aktiven Spezies über eine Sprühimprägnierung eines fluidisierten Betts von Katalysatorträgern stattfindet. Die dabei resultierenden schalenförmigen Bereiche der katalytisch aktiven Spezies weisen dabei eine weitestgehend konstante Verteilung der katalytisch aktiven Spezies auf.

In der WO 2005/065821 A1 werden Katalysatorträger mittels Aufnahme einer, die katalytisch aktiven Spezies enthaltenden Lösung in deren Porenstruktur beladen. Alternativ wird ein Gemisch aus katalytisch aktiven Spezies und Bindermaterial auf eine Oberfläche des Katalysatorträgers aufgebracht. Die Verteilung der katalytisch aktiven Spezies ist hierbei nachteilig, weil sie entweder relativ weit ins Innere des Trägers reicht oder eine äußere Schicht erzeugt wird, bei der die Verteilung über einen relativ breiten Bereich dieser aufgebrachten Schale weitestgehend homogen ist.

Es bestand weiterhin Bedarf an Pd- und Au-haltigen Schalenkatalysatoren, die sich durch ein verbessertes Profil der Pd-Konzentration auszeichnet, um so eine verbesserte katalytische Wirkung zu erzielen.

Diese Aufgabe wird durch einen Pd- und Au-haltigen Schalenkatalysator gelöst, der durch einen Konzentrationsverlauf des Palladiums innerhalb des Schalenkatalysators gekennzeichnet ist, der einer asymmetrischen Gauß-Verteilung entspricht, bei der sich das Maximum der Palladiumkonzentration in einem Bereich von 0 bis 40 Mikrometer ausgehend von der geometrischen Oberfläche des Katalysatorformkörpers befindet, das Palladium in einem Bereich mit einer Schalendicke von 30 bis 200 $\mu$m vorliegt, die Halbwertsbreite der Konzentrationsverteilung 10 bis 70 $\mu$m beträgt und die Asymmetrie der Gaußverteilung dadurch gekennzeichnet ist, dass das Verhältnis von linkem zu rechtem Halbwertsbreitenanteil im Bereich von 0,05 bis 0,55 liegt.

Ein weiterer Gegenstand der vorliegenden Erfindung ist ein erstes Verfahren zur Herstellung des erfindungsgemäßen Schalenkatalysators. Dieses umfasst folgende Schritte:

(a) Unterziehen einer Schüttung eines Katalysatorträgers einer Umwälzbewegung, wobei die Schüttung während dem Unterziehen der Umwälzbewegung auf eine Temperatur von 60°C bis 120°C erwärmt wird;
(b) Sequentielles oder gleichzeitiges Aufbringen einer gelösten Pd-haltigen Vorläuferverbindung und einer gelösten Au-haltigen Vorläuferverbindung durch Aufsprühen auf die der Umwälzbewegung unterzogenen Schüttung des Katalysatorträgers, wobei bei einer gleichzeitigen Aufbringung die Temperatur 5 bis 30 °C, bevorzugt 7 bis 25 °C, am bevorzugtesten 10 bis 20 °C unter der in Schritt (a) eingestellten Temperatur liegt und wobei bei einer sequentiellen Aufbringung die Temperatur der ersten Aufbringung 5 bis 30 °C, bevorzugt 7 bis 25 °C, am bevorzugtesten 10 bis 20 °C unter der in Schritt (a) eingestellten Temperatur liegt.

Der erfindungsgemäße Katalysator lässt sich außerdem durch ein zweites erfindungsgemäßes Verfahren herstellen. Dieses umfasst folgende Schritte:

(a) Unterziehen einer Schüttung eines Katalysatorträgers einer Umwälzbewegung und In-Kontaktbringen mit der Schüttung mit zerstäubtem Wasser durch Aufsprühen bei einer Temperatur im Bereich von 55 bis 110°C;
(b) Sequentielles oder gleichzeitiges Aufbringen einer gelösten Pd-haltigen Vorläuferverbindung und einer gelösten Au-haltigen Vorläuferverbindung durch Aufsprühen auf die der Umwälzbewegung unterzogenen Schüttung des Katalysatorträgers, wobei bei einer gleichzeitigen Aufbringung die Temperatur dieselbe wie in Schritt (a) ist, und wobei bei einer sequentiellen Aufbringung die Temperatur der ersten Aufbringung dieselbe wie in Schritt (a) ist.

Überraschenderweise wurde festgestellt, dass mittels der erfindungsgemäßen Verfahren Schalenkatalysatoren herstellbar sind, die sich durch eine verbesserte Pd-Verteilung im Inneren der einzelnen Katalysatorformkörper auszeichnen, wobei die Pd-Konzentrationsverteilung in Form einer asymmetrischen Gaußfunktion vorliegt, sich das Maximum der Pd-Konzentration in einem Bereich von 0 bis 40 Mikrometer ausgehend von der Oberfläche des Katalysatorformkörpers befindet, das Palladium in einem Bereich mit einer Schalendicke von 30 bis 200 $\mu$m vorliegt, die Halbwertsbreite der Konzentrationsverteilung 10 bis 70 $\mu$m beträgt und die Asymmetrie der Gaußverteilung dadurch gekennzeichnet ist, dass das Verhältnis von linkem zu rechtem Halbwertsbreitenanteil im Bereich von 0,05 bis 0,55 liegt.

Unter einem Schalenkatalysator im Sinne der vorliegenden Erfindung ist ein Katalysator zu verstehen, der als Pd- und Au-haltiger Katalysatorformkörper vorliegt und in dem die Pd-Verbindung und Au-Verbindung als katalytisch aktive Spezies im äußeren Bereich des Trägermaterials vorliegen und eine sogenannte Schale bilden, während das Innere des Formkörpers im Wesentlichen frei von Pd-Verbindungen und Au-Verbindungen ist. Üblicherweise weist die Schale eine hohe Konzentration der katalytisch aktiven Spezies in einem schmalen Bereich des Katalysatorformkörpers auf.

In Bezug auf die beiden erfindungsgemäßen Verfahren liegt der Schalenkatalysator nach Schritt (b) als Katalysatorformkörper vor, davor handelt es sich um einen Katalysatorträger.

Das Maximum der Pd-Konzentration im Schalenkatalysator liegt im Bereich von 0 bis 40 Mikrometern, bevorzugt im Bereich von 5 bis 40 Mikrometer, bevorzugter 5 bis 35 Mikrometer, besonders bevorzugt 5 bis 30 Mikrometer, jeweils ausgehend von der geometrischen Oberfläche des Katalysatorformkörpers.

Der Schalenkatalysator weist eine Schale umfassend Pd mit einer Dicke von 30 bis 200 Mikrometer, bevorzugt 40 bis 180 Mikrometer, besonders bevorzugt 50 bis 160 Mikrometer, am bevorzugtesten 60 bis 140 Mikrometer auf.

In einer Ausführungsform weist der Schalenkatalysator eine Halbwertsbreite der Konzentrationsverteilung des Pd im Bereich von 45 bis 65 $\mu$m auf.

In einer Ausführungsform weist der Schalenkatalysator einen Pd-Konzentrationsverlauf in Form einer asymmetrischen Gaußverteilung auf, der dadurch definiert ist, dass das Verhältnis von linkem zu rechtem Halbwertsbreitenanteil im Bereich von 0,05 bis 0,55, bevorzugt im Bereich von 0,05 bis 0,45 und besonders bevorzugt im Bereich von 0,05 bis 0,35 liegt.

Das Unterziehen einer Schüttung eines Katalysatorträgers einer Umwälzbewegung wird üblicherweise mithilfe geeigneter Fließbettanlagen durchgeführt. Solche Anlagen werden von den Unternehmen Glatt GmbH (Binzen, Deutschland), Aeromatic-Fielder AG (Bubendorf, Schweiz), Fluid Air Inc. (Aurora, Illinois, USA), Hüttlin GmbH (Steinen, Deutschland), Umang Pharmatech Pvt. Ltd. (Marharashtra, Indien) und Romaco Pharmatechnik GmbH (Karlsruhe, Deutschland) vertrieben. Zur Durchführung des erfindungsgemäßen Verfahrens besonders bevorzugte Fließbettvorrichtungen werden von der Firma Romaco Pharmatechnik GmbH unter den Bezeichnungen Innojet® Ventilus oder Innojet® AirCoater vertrieben. Diese Vorrichtungen umfassen einen zylindrischen Behälter mit einem fest und unbeweglich eingebauten Behälterboden, in dessen Mitte eine Sprühdüse montiert ist. Der Boden besteht aus kreisrunden Lamellen, die stufenweise übereinander montiert sind. Die Prozessluft strömt zwischen den einzelnen Lamellen waagerecht exzentrisch mit einer umfänglichen Strömungskomponente nach außen in Richtung der Behälterwand in den Behälter ein. Dabei bilden sich so genannte Luftgleitschichten aus, auf denen die Katalysatorträger-Formkörper, zunächst nach außen in Richtung Behälterwand transportiert werden. Außen an der Behälterwand ist ein senkrecht ausgerichteter Prozessluftstrom installiert, der die Katalysatorträger nach oben umlenkt. Oben angekommen bewegen sich die Katalysatorträger auf einer tangentialen Bahn in Richtung Mitte des Bodens zurück, in dessen Verlauf sie den Sprühnebel der Düse passieren. Nach dem Passieren des Sprühnebels beginnt der beschriebene Bewegungsvorgang von Neuem. Die beschriebene Prozessluftführung liefert dabei die Grundlage für eine weitgehend homogene toroidale fließbettartige Umwälzbewegung der Katalysatorträger.

In den erfindungsgemäßen Verfahren wird bevorzugt ein Fließbett erzeugt, in welchem die Formkörper elliptisch oder toroidal umlaufen. Im Stand der Technik wird der Übergang der Partikel einer Schüttung in einen Zustand, in welchen die Partikel vollständig frei beweglich werden (Fließbett), als Lockerungspunkt (Wirbelpunkt) bezeichnet und die entsprechende Fluidgeschwindigkeit als Lockerungsgeschwindigkeit. Erfindungsgemäß bevorzugt ist es, dass in den erfindungsgemäßen Verfahren die Fluidgeschwindigkeit bis zum 4-fachen der Lockerungsgeschwindigkeit beträgt, bevorzugt bis zum drei-fachen der Lockerungsgeschwindigkeit und mehr bevorzugt bis zum 2-fachen der Lockerungsgeschwindigkeit.

In den erfindungsgemäßen Verfahren laufen die Katalysatorträger in dem Fließbett elliptisch oder toroidal um, vorzugsweise toroidal. Unter einem "elliptischem Umlaufen" ist zu verstehen, dass sich die Katalysatorträger in dem Fließbett in vertikaler Ebene auf einer elliptischen Bahn bewegen mit wechselnder Größe der Haupt- und Nebenachse. Beim "toroidalen Umlaufen" bewegen sich die Katalysatorträger in dem Fließbett in vertikaler Ebene auf einer elliptischen Bahn mit wechselnder Größe der Haupt- und Nebenachse und in horizontaler Ebene auf einer Kreisbahn mit wechselnder Größe des Radius. Im Mittel bewegen sich die Katalysatorträger bei "elliptischem Umlaufen" in vertikaler Ebene auf einer elliptischen Bahn, bei "toroidalen Umlaufen" auf einer toroidalen Bahn, d.h., dass ein Katalysatorträger die Oberfläche eines Torus mit vertikal elliptischem Schnitt helikal abfährt.

Vor der Aufbringung der Pd- und Au-haltigen Vorläuferverbindungen wird in einer Ausführungsform ein Acetat auf die Katalysatorträger aufgebracht. Hierzu wird vorzugsweise eine Lösung enthaltend das Acetat hergestellt. Als Lösungsmittel zur Herstellung der Lösung wird vorzugsweise Wasser verwendet, stärker bevorzugt entionisiertes Wasser. Als Acetat wird üblicherweise ein Alkaliacetat verwendet, bevorzugt Kaliumacetat.

Das Aufbringen der Lösung enthaltend das Acetat kann durch jegliches im Stand der Technik bekannte Verfahren, wie beispielsweise nasschemisches Imprägnieren, Porenfüllmethode (incipient-wetness) sowie durch Sprühimprägnierung jeglicher Art durchgeführt werden.

Die Aufbringung des Acetats findet bevorzugt vor Schritt a) der erfindungsgemäßen Verfahren statt.

Liegt das Acetat als Alkaliacetat vor, so liegt die Beladung an Alkalimetall im entsprechenden stöchiometrischen Verhältnis zu den Acetatanionen.

Nach dem Aufbringen der Acetat enthaltenden Lösung wird vorzugsweise eine Trocknung im Temperaturbereich von 70 bis 120°C, stärker bevorzugt 80 bis 110°C und am stärksten bevorzugt 90 bis 100°C in Luft, Magerluft oder Inertgas durchgeführt. Die Zeitdauer für die Trocknung der mit Acetat beladenen Trägerkörper liegt vorzugsweise im Bereich von 10 bis 100 Minuten, stärker bevorzugt 30 bis 60 Minuten. Die Trocknung des mit Acetat beladenen Trägerkörpers kann in einer konventionellen Trocknungsvorrichtung aber auch in der Beschichtungsvorrichtung durchgeführt werden. Wird die Trocknung in der Beschichtungsvorrichtung durchgeführt, so wird vorzugsweise so getrocknet, dass die Trägerkörper statisch darin vorliegen, d.h., dass sie nicht bewegt werden. Im Falle der Verwendung einer Fließbett- oder Wirbelschichtvorrichtung werden die Trägerkörper während der Trocknung vorzugsweise nicht im Fließbett oder der Wirbelschicht bewegt.

In Schritt (b) werden eine gelöste Pd-Vorläuferverbindung und eine gelöste Au-Vorläuferverbindung auf die der Umwälzbewegung unterzogene Schüttung des Katalysatorträgers aufgebracht.

Beispiele für bevorzugte Pd-Vorläuferverbindungen sind wasserlösliche Pd-Salze. Besonders bevorzugt ist die Pd-Vorläuferverbindung ausgewählt aus der Gruppe bestehend aus $Pd(NH_3)_4(HCO_3)_2$, $Pd(NH_3)_4(HPO_4)$, Ammonium-Pd-Oxalat, Pd-Oxalat, $K_2Pd(Oxalat)_2$, Pd(II)-Triflouracetat, $Pd(NH_3)_4(OH)_2$, $Pd(NO_3)_2$, $H_2Pd(OAc)_2(OH)_2$, $Pd(NH_3)_2(NO_2)_2$, $Pd(NH_3)_4(NO_3)_2$, $H_2Pd(NO_2)_4$, $Na_2Pd(NO_2)_4$, $Pd(OAc)_2$ sowie frisch ausgefälltes $Pd(OH)_2$.

Wird frisch ausgefälltes $Pd(OH)_2$ verwendet, dann wird dieses vorzugsweise folgendermaßen hergestellt: Es wird vorzugsweise eine 0,1 bis 40 Gew.-%ige wässrige Lösung aus Tetrachloropalladat hergestellt. Dann wird vorzugsweise zu dieser Lösung eine Base hinzugegeben, bis ein brauner Feststoff, nämlich das $Pd(OH)_2$ ausfällt. Zur Herstellung einer Lösung zum Auftragen auf den Katalysatorträger wird das frisch ausgefällte $Pd(OH)_2$ isoliert, gewaschen und in einer wässrig-alkalischen Lösung gelöst.

Die Verbindung $Pd(NH_3)_4(OH)_2$ wird vorzugsweise folgendermaßen hergestellt: Eine Vorläuferverbindung wie z.B. $Na_2PdCl_4$ wird - wie zuvor beschrieben - mit Kalilauge zu Palladiumhydroxid gefällt und der Niederschlag, nach Filtration und Waschung, in wässrigen Ammoniak zu $Pd(NH_3)_4(OH)_2$ gelöst.

Weiterhin können in dem erfindungsgemäßen Verfahren auch Pd-Nitrit-Verbindungen eingesetzt werden. Bevorzugte Pd-Nitrit-Vorläuferverbindungen sind beispielsweise solche, die mittels Lösen von $Pd(OAc)_2$ in einer $NaNO_2$- oder $KNO_2$-Lösung erhalten werden.

Beispiele für bevorzugte Au-Vorläuferverbindungen sind wasserlösliche Au-Salze. Gemäß einer besonders bevorzugten Ausführungsform des erfindungsgemäßen Verfahrens ist die Au-Vorläuferverbindung ausgewählt aus der Gruppe bestehend aus $KAuO_2$, $NaAuO_2$, $CsAuO_2$, $NMe_4AuO_2$, $KAuCl_4$, $(NH_4)AuCl_4$, $HAuCl_4$, $KAu(NO_2)_4$, $NaAu(NO_2)_4$, $CsAu(NO_2)_4$, $AuCl_3$, $NaAuCl_4$, $CsAuCl_4$, $KAu(OAc)_3(OH)$, $NaAu(OAc)_3(OH)$, $CsAu(OAc)_3(OH)$, $HAu(NO_3)_4$ und $Au(OAc)_3$. Dabei ist es gegebenenfalls empfehlenswert, das $Au(OAc)_3$ oder das $KAuO_2$ mittels Fällung des Oxids/Hydroxids aus einer Goldsäure-Lösung, Waschung und Isolierung des Niederschlags sowie Aufnahme desselben in Essigsäure bzw. KOH jeweils frisch anzusetzen.

Unter gelösten Pd-haltigen Vorläuferverbindungen und Au-haltigen Vorläuferverbindungen im Rahmen der vorliegenden Erfindung sind solche Verbindungen zu verstehen, die in einem Lösungsmittel, bevorzugt Wasser, wässriger Base oder wässriger Säure, gelöst vorliegen.

Im Rahmen der vorliegenden Erfindung werden solche Salze als wasserlöslich bezeichnet, die in Wasser, wässriger Base oder wässriger Säure ausreichend löslich sind.

Bevorzugt werden die Pd- und Au-Vorläuferverbindungen so gewählt, dass sie keine Katalysatorgifte enthalten und die Katalysatorformkörper nach der Aufbringung der Edelmetallverbindungen lediglich getrocknet werden, bevorzugt in demselben Wirbelschichtcoater, in dem auch die Aufbringung stattgefunden hat. Besonders bevorzugt sind die Vorläuferverbindungen im Wesentlichen chlorid-frei. Unter im Wesentlichen chlorid-frei versteht man, dass die Summenformel der Verbindung kein Chlorid umfasst, es jedoch nicht ausgeschlossen ist, dass die Verbindung beispielsweise herstellungsbedingt unvermeidliche Verunreinigungen an Chlorid enthält. Der Gehalt an Chlorid in im Wesentlichen chlorid-freien Pd- und Au-Vorläuferverbindungen beträgt höchstens 125 ppm pro Gewichts-% Pd und Au in der Vorläuferverbindung.

Die Aufbringung der Pd-haltigen Vorläuferverbindung und der Au-haltigen Vorläuferverbindung erfolgt durch sequentielles, d.h. nacheinander erfolgendes, oder gleichzeitiges Aufbringen einer gelösten Pd-haltigen Vorläuferverbindung und einer gelösten Au-haltigen Vorläuferverbindung durch Aufsprühen auf die der Umwälzbewegung unterzogenen Schüttung des Katalysatorträgers. Diese Aufbringung kann durch gleichzeitiges Aufbringen einer Lösung einer Pd-haltigen Vorläuferverbindung und einer Lösung einer Au-haltigen Vorläuferverbindung erfolgen. Sie kann auch durch gleichzeitiges Aufbringen einer Lösung einer Pd-haltigen Vorläuferverbindung und einer Au-haltigen Vorläuferverbindung erfolgen. Sie kann auch durch sequentielles Aufbringen erfolgen, wobei in einem ersten Schritt eine Lösung einer Pd-haltigen Vorläuferverbindung und in einem zweiten Schritt eine Lösung einer Au-haltigen Vorläuferverbindung erfolgt oder in einem ersten Schritt eine Lösung einer Au-haltigen Vorläuferverbindung und in einem zweiten Schritt eine Lösung einer Pd-haltigen Vorläuferverbindung erfolgt.

In einer Ausführungsform enthält die bei sequentieller Aufbringung im zweiten Schritt aufgebrachte Lösung sowohl eine Pd-haltige Vorläuferverbindung als auch eine Au-haltige Vorläuferverbindung.

Soll der Schalenkatalysator in der Schale mehrere voneinander verschiedene katalytisch aktive Spezies enthalten, beispielsweise mehrere Aktivmetalle oder ein Aktivmetall und ein Promotormetall, so kann der Katalysatorträger-Formkörper dem erfindungsgemäßen Verfahren entsprechend häufig unterworfen werden. Alternativ dazu kann das erfindungsgemäße Verfahren auch mit Mischlösungen durchgeführt werden, welche die gewünschten, voneinander verschiedenen katalytisch aktiven Spezies oder Vorläufer davon enthalten. Ferner können gemäß dem erfindungsgemäßen Verfahren die Katalysatorträger gleichzeitig mit voneinander verschiedenen Lösungen verschiedener katalytisch aktiver Spezies oder Vorläufer davon besprüht werden.

Bei dem in den erfindungsgemäßen Verfahren verwendeten Prozessgas handelt es sich üblicherweise um Luft. In einer weiteren Ausführungsform kann auch ein Inertgas, beispielsweise Stickstoff, Methan, kurzkettige gesättigte Kohlenwasserstoffe, ein Edelgas, vorzugsweise Helium, Neon oder Argon, oder ein halogenierter Kohlenwasserstoff oder eine Mischung von zwei oder mehr der vorgenannten, verwendet werden.

Durch die gezielte Erwärmung des Katalysatorträgers in Schritt (a) und in Schritt b) des ersten erfindungsgemäßen Verfahrens kann die Breite der ausgebildeten Edelmetallschale kontrolliert werden, da je nach Temperatur bei gemeinsamer Aufbringung der Pd-haltigen Vorläuferverbindung und Au-haltigen Vorläuferverbindung eine schnellere Verdunstung der in Schritt b) aufgebrachten mindestens einen, bevorzugt wässrigen Lösung erfolgt oder bei sequentieller Aufbringung der Pd-haltigen Vorläuferverbindung und Au-haltigen Vorläuferverbindung eine schnellere Verdunstung der in Schritt b) aufgebrachten ersten aufgebrachten, bevorzugt wässrigen Lösung erfolgt und somit die Diffusion der Edelmetalle ins Trägerinnere verringert wird. Bei relativ hohen Temperaturen beispielsweise ist die Abtrocknungsgeschwindigkeit verhältnismäßig hoch, so dass mit dem Katalysatorträger in Kontakt kommende Lösung nahezu unverzüglich abtrocknet, weshalb auf dem Katalysatorträger aufgetragene Lösung nicht tief in denselben eindringen kann. Bei verhältnismäßig hohen Temperaturen können so Schalen mit relativ kleinen Dicken und hoher Beladung an aktiver Spezies erhalten werden.

Es wurde überraschend gefunden, dass in dem ersten erfindungsgemäßen Verfahren durch die in Schritt (a) durchgeführte Erwärmung des Katalysatorträgers auf eine Temperatur, die 5 bis 30 °C, bevorzugt 7 bis 25 °C, am bevorzugtesten 10 bis 20 °C oberhalb der Temperatur der Aufbringung, bzw. im Falle einer sequentiellen Aufbringung der Temperatur der ersten Aufbringung in Schritt (b) ist, die Verteilung des Pd vorteilhaft kontrolliert werden kann, indem sich das Maximum der Pd-Konzentration in einem Bereich von 0 bis 40 Mikrometer ausgehend von der Oberfläche des Katalysatorformkörpers befindet, das Palladium in einem Bereich mit einer Schalendicke von 30 bis 200 $\mu$m vorliegt und die Halbwertsbreite der Konzentrationsverteilung 10 bis 70 $\mu$m beträgt.

Die in Schritt (a) bzw. (b) einzustellende Temperatur des Katalysatorträgers wird dabei kontrolliert, indem die Ablufttemperatur bzw. Temperatur der Schüttung gemessen und ggf. nachgesteuert wird.

Die in Schritt (a) einzustellende Temperatur liegt im Bereich von 60 bis 120 °C, bevorzugt von 65 bis 110 °C, besonders bevorzugt 70 bis 100 °C.

Die in Schritt (b) einzustellende Temperatur der Aufbringung, bzw. im Falle einer sequentiellen Aufbringung der Temperatur der ersten Aufbringung liegt im Bereich von 55 bis 115 °C, bevorzugt im Bereich von 55 bis 110 °C, mehr bevorzugt im Bereich von 60 bis 100 °C, besonders bevorzugt im Bereich von 65 bis 90 °C. Bei einer sequentiellen Aufbringung kann die zweite Aufbringung bei derselben Temperatur wie die erste Aufbringung erfolgen. In einer Ausführungsform ist die Temperatur während der zweiten Aufbringung 5 bis 30 °C, bevorzugt 5 bis 20 °C, am bevorzugtesten 5 bis 10 °C höher als während der ersten Aufbringung.

Durch das Aufsprühen mit Wasser in Schritt a) des zweiten erfindungsgemäßen Verfahrens kann die Breite der ausgebildeten Edelmetallschale im gleichen Maße kontrolliert werden, da hierbei das Wasser bei In-Kontakt-Bringen mit dem Katalysatorträger verdunstet und zu einer vorübergehenden Temperaturerniedrigung des Katalysatorträgers führt, die durch die Temperaturregelung der Beschichtungsapparatur wieder ausgeglichen wird, so dass die anschließende Aufbringung der metallhaltigen wässrigen Lösungen bei der gewünschten Zieltemperatur erfolgen kann, womit eine schnellere Verdunstung der in Schritt b) aufgebrachten wässrigen Lösung, bzw. im Falle einer sequentiellen Aufbringung eine schnellere Verdunstung der ersten aufgebrachten Lösung möglich ist und somit die Diffusion der Edelmetalle ins Trägerinnere verringert wird. Bei relativ hohen Temperaturen beispielsweise ist die Abtrocknungsgeschwindigkeit verhältnismäßig hoch, so dass mit dem Katalysatorträger in Kontakt kommende Lösung nahezu unverzüglich abtrocknet, weshalb auf dem Katalysatorträger aufgetragene Lösung nicht tief in denselben eindringen kann. Bei verhältnismäßig hohen Temperaturen können so Schalen mit relativ kleinen Dicken und hoher Beladung an aktiver Spezies erhalten werden.

Die in Schritt (a) bzw. (b) einzustellende Temperatur des Katalysatorträgers wird dabei kontrolliert, indem die Ablufttemperatur bzw. Temperatur der Schüttung gemessen und ggf. nachgesteuert wird.

Die in Schritt (a) einzustellende Temperatur liegt im Bereich von 55 bis 110 °C, bevorzugt von 60 bis 100 °C, besonders bevorzugt 65 bis 90 °C.

Die in Schritt (b) einzustellende Temperatur der Aufbringung, bzw. im Falle einer sequentiellen Aufbringung der Temperatur der ersten Aufbringung ist dieselbe wie in Schritt (a) und liegt im Bereich von 55 bis 110 °C, bevorzugt von 60 bis 100 °C, besonders bevorzugt 65 bis 90 °C. Bei einer sequentiellen Aufbringung kann die zweite Aufbringung bei derselben Temperatur wie die erste Aufbringung erfolgen. In einer Ausführungsform ist die Temperatur während der zweiten Aufbringung 5 bis 30 °C, bevorzugt 5 bis 20 °C, am bevorzugtesten 5 bis 10 °C höher.

Die Trocknung der mit der Lösung besprühten Katalysatorträger erfolgt im Rahmen des erfindungsgemäßen Verfahrens vorzugsweise kontinuierlich mittels des Prozessgases.

In einer Ausführungsform der erfindungsgemäßen Verfahren wird der Katalysatorträger nach Schritt (b) einem Fixierungsschritt unterworfen wird, um die katalytisch aktiven Spezies oder deren Vorläufer auf dem Katalysatorträger zu fixieren. Geeignete Maßnahmen stellen das Besprühen der mit den Edelmetallverbindungen beladenen Katalysatorträger mit einer Lauge oder das Tauchen der Katalysatorträger in eine laugenhaltige Lösung dar. Im Anschluss erfolgt eine Behandlung mit Wasser, um überschüssige Lauge vom Katalysatorträger zu entfernen. Dieses Verfahren eignet sich insbesondere, wenn die aufgebrachte Pd- oder ggf. Au-Vorläuferverbindung Bestandteile enthält, die als Katalysatorgifte, insbesondere bei der Herstellung von VAM, gelten, wie zum Beispiel die entsprechenden Pd- oder Au-Chloride. In einer weiteren Ausführungsform wird der nach Schritt (b) erhaltene Katalysatorträger bei einer Temperatur im Bereich von 300 °C bis 600 °C thermisch behandelt, für insbesondere 1 h bis 6 h, um die Vorläuferverbindungen in die entsprechenden Hydroxid-Verbindungen bzw. Oxide zu überführen. In einer weiteren Ausführungsform wird der nach Schritt (b) erhaltene Katalysatorträger bei einer Temperatur im Bereich von 80 bis 200 °C, bevorzugt 100 bis 150 °C getrocknet.

In einer weiteren Ausführungsform wird der nach Schritt (b) erhaltene Katalysatorträger keinem Fixierungsschritt unterworfen, aber bei einer Temperatur im Bereich von 80 bis 200 °C, bevorzugt 100 bis 150 °C getrocknet.

Besonders bevorzugt ist es jedoch, dass weder ein Schritt der Trocknung noch der thermischen Behandlung im Bereich von 300 °C und 600 °C nach dem Schritt (b) und vor der Reduktion in Schritt (c) erfolgt.

In einer Ausführungsform findet nach Schritt (b) in einem Schritt (c) eine Reduktion der Metallkomponenten der Vorläuferverbindungen zu den elementaren Metallen durch Unterziehen des nach Schritt (b) erhaltenen Katalysatorformkörpers mittels einer Temperaturbehandlung in einer nicht-oxidierenden Atmosphäre

Die Reduktion in Schritt (c) kann sowohl in dem Wirbelschichtcoater selbst oder in einem separaten Reduktionsreaktor stattfinden. Findet die Reduktion in dem Wirbelschichtcoater statt, wird dies üblicherweise mit einer Mischung aus 2 bis 5 Vol.% Wasserstoff in Stickstoff bei einer Temperatur im Bereich von 70 bis 150 °C über einen Zeitraum von beispielsweise 30 min bis 5 Stunden durchgeführt. Wenn die Reduktion in einem separaten Reduktionsreaktor durchgeführt wird, wird dies üblicherweise mit einer Mischung aus 2 bis 5 Vol.-% Wasserstoff in Stickstoff, zum Beispiel einem Formiergas, bei Temperaturen im Bereich von bevorzugt 70 -500 °C über einen Zeitraum von 30 min bis 5 Stunden durchgeführt.

Als weitere Reduktionsmittel eignen sich Ethylen, CO, $NH_3$, Formaldehyd, Methanol und Kohlenwasserstoffe, wobei die Reduktionsmittel auch mit einem Inertgas, wie beispielsweise Kohlendioxid, Stickstoff oder Argon, verdünnt sein können. Vorzugsweise wird ein mit Inertgas verdünntes Reduktionsmittel eingesetzt. Bevorzugt sind Mischungen von Wasserstoff mit Stickstoff oder Argon, vorzugsweise mit einem Wasserstoffgehalt zwischen 1 Vol.-% und 15 Vol.-%.

In einer weiteren Ausführungsform kann die Reduktion der Edelmetalle in einer reinen Stickstoffatmosphäre bei einer Temperatur im Bereich von 130 bis 200 °C, bevorzugt im Bereich von 140 bis 170 °C durchgeführt werden.

In einer weiteren Ausführungsform kann die Reduktion der Edelmetalle auch in flüssiger Phase erfolgen, bevorzugt unter Verwendung der Reduktionsmittel Hydrazin, K-Formiat, Na-Formiat, Ameisensäure, $H_2O_2$, hypophosphorige Säure oder Na-hypophosphit.

Der in den erfindungsgemäßen Verfahren verwendete Katalysatorträger kann verschiedene Formen aufweisen, beispielsweise Tabletten, Zylinder, Ringe, irreguläre Granulate oder Kugeln. Bevorzugt weist der Katalysatorträger eine Kugelform auf. Dessen Geometrie ermöglicht in der Umwälzbewegung eine gleichmäßige Rotation des Trägers um seine Achse und daraus resultierend eine gleichmäßige Imprägnierung des Katalysatorträgers mit der Lösung der katalytisch aktiven Spezies. Bei dem in dem erfindungsgemäßen Verfahren verwendeten Katalysatorträger handelt es sich nicht um ein pulverförmiges Material.

Der Katalysatorträger in Kugelform weist einen arithmetischen Durchmesser im Bereich von 1 bis 10 mm, bevorzugt 3 bis 9, besonders bevorzugt 3 bis 8 mm auf. Unter einer Kugelform im Rahmen der vorliegenden Anmeldung wird auch ein rotationsellipsoider Körper verstanden, wobei der Durchmesser entlang der Achse von Pol zu Pol kleiner ist als der Durchmesser entlang der entsprechenden Äquatorachse. Das Verhältnis zwischen dem Durchmesser entlang der Achse von Pol zu Pol und dem Durchmesser entlang der Äquatorachse liegt hierbei im Bereich von 0,9 bis 1,0. Der arithmetische Durchmesser dieser weitestgehend kugelförmigen Körper wir anhand der Durchmesser von Pol zu Pol bestimmt.

Der Katalysatorträger umfasst üblicherweise eine Verbindung aus der Gruppe von Titanoxid, Siliciumoxid, Aluminiumoxid, Zirkoniumoxid, Magnesiumoxid, Siliciumcarbid, Magnesiumsilikat, Zinkoxid, Zeolithe, Schichtsilikate oder Mischungen davon. Vorzugsweise umfasst der Katalysatorträger ein Si-Al-Mischoxid, insbesondere in Form eines Schichtsilikats, vorzugsweise ein Schichtsilikat in Form eines kalzinierten säurebehandelten Bentonits. Der Anteil dieser Verbindungen bzw. deren Mischungen beträgt üblicherweise mindestens 70 Gew.-%, bevorzugt mindestens 80 Gew.-%, besonders bevorzugt mindestens 90 Gew.-%, bezogen auf das Gewicht des Katalysatorträgers nach Glühverlust. In einer besonders bevorzugten Ausführungsform umfasst der Katalysatorträger einen kalzinierten säurebehandelten Bentonit in einem Anteil von mindestens 70 Gew.-%, bevorzugt mindestens 80 Gew.-%, besonders bevorzugt mindestens 90 Gew.-% und am bevorzugtesten mindestens 95 Gew.-% bezogen auf das Gewicht des Katalysatorträgers nach Glühverlust.

Unter dem Begriff "natürliches Schichtsilikat", wofür in der Literatur auch der Begriff "Phyllosilikat" verwendet wird, wird aus natürlichen Quellen stammendes unbehandeltes oder behandeltes Silikat-Mineral verstanden, in welchem $SiO_4$-Tetraeder in Schichten der allgemeinen Formel $[Si_2O_5]^{2-}$ miteinander vernetzt sind. Diese Tetraederschichten wechsellagern mit so genannten Oktaederschichten, in denen ein Kation, vor allem Al und Mg, oktaedrisch von OH bzw. O umgeben ist. Dabei werden beispielsweise Zweischicht-Phyllosilikate und Dreischicht-Phyllosilikate unterschieden. Im Rahmen der vorliegenden Erfindung bevorzugte Schichtsilikate sind Tonminerale, insbesondere Kaolinit, Beidellit, Hectorit, Saponit, Nontronit, Glimmer, Vermiculit und Smektite, wobei Smektite und dabei insbesondere Montmorillonit besonders bevorzugt sind. Definitionen des Begriffes "Schichtsilikate" finden sich beispielsweise in "Lehrbuch der anorganischen Chemie", Hollemann Wiberg, de Gruyter, 102. Auflage, 2007 (ISBN 978-3-11-017770-1) oder in "Römpp Lexikon Chemie", 10. Auflage, Georg Thieme Verlag unter dem Begriff "Phyllosilikat". Typische Behandlungen, denen ein natürliches Schichtsilikat vor dem Einsatz als Trägermaterial unterzogen wird, beinhalten beispielsweise ein Behandeln mit Säuren und/oder ein Kalzinieren. Ein im Rahmen der vorliegenden Erfindung besonders bevorzugtes natürliches Schichtsilikat ist ein Bentonit. Bentonite sind zwar im eigentlichen Sinne keine natürlichen Schichtsilikate, sondern vielmehr ein Gemisch von überwiegend Tonmineralien, in welchem Schichtsilikate enthalten sind. Vorliegend ist für den Fall, dass das natürliche Schichtsilikat ein Bentonit ist, zu verstehen, dass das natürliche Schichtsilikat in dem Katalysatorträger in Form oder als Bestandteil eines kalzinierten säurebehandelten Bentonits vorliegt.

Ein als Formkörper ausgebildeter Katalysatorträger auf der Basis von natürlichen Schichtsilikaten, insbesondere auf der Basis eines säurebehandelten kalzinierten Bentonits, kann beispielsweise hergestellt werden, indem eine einen säurebehandelten (unkalzinierten) Bentonit und Wasser enthaltende Mischung unter Verdichtung zu einem Formkörper mittels dem Fachmann geläufiger Vorrichtungen, wie beispielsweise Extrudern oder Tablettenpressen, geformt wird und anschließend der nicht ausgehärtete Formkörper zu einem stabilen Formkörper kalziniert wird. Dabei hängt die Größe der spezifischen Oberfläche des Katalysatorträgers insbesondere von der Qualität des eingesetzten (Roh-)Bentonits ab, dem Säurebehandlungsverfahren des eingesetzten Bentonits, d.h. beispielsweise der Natur und der zum Bentonit relativen Menge und der Konzentration der eingesetzten anorganischen Säure, der Säurebehandlungsdauer sowie der -temperatur, vom Verpressungsdruck sowie von der Kalzinierdauer und -temperatur sowie der Kalzinieratmosphäre.

Säurebehandelte Bentonite können durch Behandlung von Bentoniten mit starken Säuren erhalten werden, wie beispielsweise Schwefelsäure, Phosphorsäure oder Salzsäure. Eine auch im Rahmen der vorliegenden Erfindung geltende Definition des Begriffes Bentonit ist in Römpp, Lexikon Chemie, 10. Aufl., Georg Thieme Verlag, angegeben. Im Rahmen der vorliegenden Erfindung besonders bevorzugte Bentonite sind natürliche aluminiumhaltige Schichtsilikate, die Montmorillonit als Hauptmineral enthalten. Nach der Säurebehandlung wird der Bentonit in der Regel mit Wasser gewaschen, getrocknet und zu einem Pulver vermahlen.

Die BET-Oberfläche des Katalysatorträgers beträgt 10 bis 600 m$^2$/g, bevorzugt 20 bis 400 m$^2$/g und besonders bevorzugt zwischen 80 und 170 m$^2$/g. Die BET-Oberfläche wird nach der 1-Punkt-Methode durch Adsorption von Stickstoff nach DIN 66132 bestimmt.

In einer Ausführungsform beträgt das integrale Porenvolumen des Katalysatorträgers (bestimmt gemäß DIN 66133 (Hg-Porosimetrie)) ohne die Beschichtung mit der Vorläuferverbindung mindestens 0,1 ml/g, vorzugsweise mindestens 0,18 ml/g, mehr bevorzugt mindestens 0,4 ml/g. In einer weiteren Ausführungsform liegt das integrale Porenvolumen im Bereich von 0,1 ml/g bis 0,8 ml/g, bevorzugt im Bereich von 0,18 ml/g bis 0,6 ml/g und besonders bevorzug im Bereich von 0,35 bis 0,55 ml/g.

Der in den erfindungsgemäßen Verfahren verwendete Katalysatorträger weist eine Härte von mindestens 20 N auf, bevorzugt von mindestens 30 N, besonders bevorzugt von mindestens 40 N und am bevorzugtesten von mindestens 50 N.

Der Anteil an Pd im erfindungsgemäßen Katalysator liegt im Bereich von 0,2 bis 2,0 Gew.-%, bevorzugt im Bereich von 0,4 bis 1,75 Gew.-% und besonders bevorzugt im Bereich von 0,7 bis 1,5 Gew.-%, bezogen auf die Masse des mit Edelmetall beladenen und reduzierten Katalysatorformkörpers nach Trockenverlust.

Der Anteil an Au im erfindungsgemäßen Katalysator liegt im Bereich von 0,1 bis 1,2 Gew.-%, bevorzugt im Bereich von 0,2 bis 1,0 Gew.-% und am bevorzugtesten im Bereich von 0,3 bis 0,8 Gew.-%, bezogen auf das Gesamtgewicht des Katalysatorformkörpers nach Reduktion und Trockenverlust.

In einer bevorzugten Ausführungsform liegt das Au/Pd-Atomverhältnis des erfindungsgemäßen Katalysatorformkörpers im Bereich von 0,01 bis 1,2, bevorzugt im Bereich von 0,05 bis 1,0, bevorzugter im Bereich von 0,1 bis 0,8 und besonders bevorzugt im Bereich von 0,15 bis 0,6.

Der erfindungsgemäße Schalenkatalysator enthält bevorzugt eine Alkalimetallverbindung als Promotor, bevorzugt eine Kalium-, eine Natrium-, eine Cäsium- oder eine Rubidiumverbindung, besonders bevorzugt eine Kaliumverbindung. Zu den geeigneten und besonders bevorzugten Kaliumverbindungen gehören Kaliumacetat KOAc, Kaliumcarbonat $K_2CO_3$, Kaliumhydrogencarbonat $KHCO_3$ und Kaliumhydroxid KOH, bevorzugt Kaliumacetat KOAc. Die Kaliumverbindung kann sowohl vor als auch nach der Reduktion der Metall-Komponenten zu den Metallen Pd und Au auf den Katalysatorträger bzw. Katalysatorformkörper aufgetragen werden. In einer bevorzugten Ausführungsform wird die Kaliumverbindung vor der Aufbringung der Edelmetallverbindungen aufgebracht, besonders bevorzugt vor Schritt (a). In diesem Fall muss darauf geachtet werden, dass in den nachfolgenden Schritten wie der Aufbringung der Vorläuferverbindungen, einer eventuellen Fixierung oder der Reduktion eine thermische Behandlung nur insofern stattfindet, als diese nicht oder nur in geringem Ausmaß zur Zersetzung der Kaliumverbindung in das entsprechende Oxid führt, da sich das Kaliumoxid unter den Reaktionsbedingungen der VAM-Reaktion nur unzureichend in Kaliumacetat umwandelt. Bevorzugt wird der Katalysatorträger bzw. Katalysatorformkörper nur Temperaturen unterhalb von 200 °C ausgesetzt.

Wenn der erfindungsgemäße Katalysatorformkörper ein Alkalimetallacetat, bevorzugt Kaliumacetat, umfasst, beträgt der Gehalt des Alkalimetallacetats in der Katalysatorschüttung 0,1 bis 0,7 mol/l, bevorzugt 0,3 bis 0,5 mol/l.

In einer bevorzugten Ausführungsform des erfindungsgemäßen Katalysatorformkörpers liegt das Alkalimetall/Pd-Atomverhältnis im Bereich von 1 bis 16, bevorzugter im Bereich von 2 bis 13 und besonders bevorzugt im Bereich von 3 bis 10. Dabei ist das Alkalimetall/Pd-Atomverhältnis bevorzugt umso geringer, je kleiner die Oberfläche des Katalysatorformkörpers ist.

Im Hinblick auf eine geringe Porendiffusionslimitierung kann gemäß einer weiteren bevorzugten Ausführungsform des erfindungsgemäßen Pd/Au-Katalysators vorgesehen sein, dass der Katalysatorformkörper einen mittleren Porendurchmesser von 8 bis 50 nm aufweist, vorzugsweise einen von 10 bis 35 nm und bevorzugt einen von 11 bis 30 nm.

Die Azidität des Katalysatorformkörpers kann die Aktivität des erfindungsgemäßen Katalysators vorteilhaft beeinflussen. Entsprechend einer weiter bevorzugten Ausführungsform des erfindungsgemäßen Katalysators besitzt der Katalysatorformkörper eine Azidität im Bereich von 1 und 150 μval/g, vorzugsweise im Bereich von 5 und 130 μval/g und besonders bevorzugt im Bereich von 10 und 100 μval/g. Die Azidität des Katalysatorformkörpers wird dabei wie folgt bestimmt: 1 g des fein gemahlenen Katalysatorformkörpers wird mit 100 ml Wasser (mit einem pH-Blindwert) versetzt und unter Rühren 15 Minuten extrahiert. Anschließend wird mit 0,01 n NaOH-Lösung zumindest bis pH 7,0 titriert, wobei die Titration stufenweise erfolgt; und zwar wird zunächst 1 ml der NaOH-Lösung zu dem Extrakt getropft (1 Tropfen/Sekunde), dann 2 Minuten gewartet, der pH-Wert abgelesen, erneut 1 ml NaOH zugetropft, usw. Der Blindwert des eingesetzten Wassers wird bestimmt und die Azidität-Berechnung entsprechend korrigiert.

Die Titrationskurve (ml 0,01 NaOH gegen pH-Wert) wird dann aufgetragen und der Schnittpunkt der Titrationskurve bei pH 7 bestimmt. Berechnet werden die Moläquivalente in 10-6 äquiv/g Träger, die sich aus dem NaOH-Verbrauch für den Schnittpunkt bei pH 7 ergeben.

$$\text{Gesamtsäure:} \frac{10 \cdot ml\, 0{,}01\, n\, NaOH}{Katalysatorträger} = \mu val/g$$

Der Schalenkatalysator liegt bevorzugt kugelförmig oder nahezu kugelförmig vor. Die Kugel weist einen Durchmesser im Bereich von 1 bis 10 mm, bevorzugt 3 bis 9, besonders bevorzugt 3 bis 8 mm auf.

Zur Erhöhung der Aktivität des erfindungsgemäßen Pd/Au-Katalysators kann es vorgesehen sein, dass der Katalysatorformkörper mit zumindest einem Oxid eines Metalls dotiert ist, ausgewählt aus der Gruppe bestehend aus Zr, Hf, Ti, Nb, Ta, W, Mg, Re, Y und Fe, vorzugsweise mit $ZrO_2$, $HfO_2$ oder $Fe_2O_3$. Dabei kann es bevorzugt sein, wenn der Anteil des Katalysatorformkörper an Dotierungsoxid zwischen 0,1 und 20 Mass.-% beträgt, vorzugsweise 1,0 bis 18 Mass.-% und bevorzugt 4 bis 16 Mass.-% bezogen auf die Masse des Katalysatorformkörpers.

Gemäß einer weiter bevorzugten Ausführungsform des erfindungsgemäßen Katalysatorformkörpers kann es vorgesehen sein, wenn die Wassersaugfähigkeit des Katalysatorformkörpers 40 bis 75 % beträgt, bevorzugt 50 bis 70 % berechnet als Gewichtszunahme durch Wasseraufnahme. Die Saugfähigkeit wird bestimmt, indem 10 g des Katalysatorformkörpers mit entionisiertem Wasser 30 min lang getränkt wird, bis von der Probe keine Gasblasen mehr entweichen. Dann wird das überschüssige Wasser dekantiert und die getränkte Probe mit einem Baumwolltuch abgetupft zur Befreiung der Probe von anhaftender Feuchtigkeit. Anschließend wird der wasserbeladene Katalysatorformkörper ausgewogen und die Saugfähigkeit berechnet gemäß:

(Auswaage (g) - Einwaage (g)) x 10 = Wassersaugfähigkeit (%)

Die BET-Oberfläche des Schalenkatalysators beträgt 10 bis 600 $m^2$/g, bevorzugt 20 bis 400 $m^2$/g und besonders bevorzugt zwischen 80 und 170 $m^2$/g. Die BET-Oberfläche wird nach der 1-Punkt-Methode durch Adsorption von Stickstoff nach DIN 66132 bestimmt.

In einer Ausführungsform beträgt das integrale Porenvolumen des Schalenkatalysators (bestimmt gemäß DIN 66133 (Hg-Porosimetrie)) ohne die Beschichtung mit der Vorläuferverbindung mindestens 0,1 ml/g, vorzugsweise mindestens 0,18 ml/g, mehr bevorzugt mindestens 0,4 ml/g. In einer weiteren Ausführungsform liegt das integrale Porenvolumen im Bereich von 0,1 ml/g und 0,8 ml/g, bevorzugt im Bereich von 0,18 ml/g und 0,6 ml/g und besonders bevorzug im Bereich von 0,35 und 0,55 ml/g.

Der Schalenkatalysator weist eine Härte von mindestens 20 N auf, bevorzugt von mindestens 30 N, besonders bevorzugt von mindestens 40 N und am bevorzugtesten von mindestens 50 N.

Gemäß einer weiter bevorzugten Ausführungsform des erfindungsgemäßen Katalysatorformkörpers kann es bevorzugt sein, wenn zumindest 80 % des integralen Porenvolumens des Katalysatorformkörpers von Mesoporen und Makroporen gebildet sind, vorzugsweise zumindest 85 % und bevorzugt zumindest 90 %. Dadurch wird einer durch Diffusionslimitierung bewirkten verminderten Aktivität des erfindungsgemäßen Katalysatorformkörpers entgegengewirkt, insbesondere bei Schalen mit verhältnismäßig großen Dicken. Dabei sollen diesbezüglich unter den Begriffen Mikroporen, Mesoporen und Makroporen Poren verstanden werden, die einen Durchmesser von kleiner als 2 nm, einen Durchmesser im Bereich von 2 bis 50 nm bzw. einen Durchmesser von größer als 50 nm aufweisen.

Ein weiterer Gegenstand der vorliegenden Erfindung betrifft ein Verfahren zur Herstellung von Alkenylcarbonsäureestern, insbesondere VAM oder Allylacetatmonomer mit dem erfindungsgemäßen Schalenkatalysator. Das Verfahren zur Herstellung von VAM wird durchgeführt, indem Essigsäure, Ethylen und Sauerstoff oder Sauerstoff enthaltende Gase über den erfindungsgemäßen Katalysatorformkörper geleitet werden. Dies erfolgt im Allgemeinen durch Leiten von Essigsäure, Ethylen und Sauerstoff oder Sauerstoff enthaltenden Gasen bei Temperaturen im Bereich von 100 bis 200°C, vorzugsweise im Bereich von 120 bis 200°C, und bei Drücken im Bereich von 1 bis 25 bar, bevorzugt im Bereich von 1 bis 20 bar, über den erfindungsgemäßen Katalysatorformkörper, wobei nicht umgesetzte Edukte im Kreis geführt werden können. Zweckmäßig hält man die Sauerstoffkonzentration unter 10 Vol.-%. Unter Umständen ist jedoch auch eine Verdünnung mit inerten Gasen wie Stickstoff oder Kohlendioxid vorteilhaft. Besonders Kohlendioxid eignet sich zur Verdünnung, da es im Zuge der VAM-Synthese in geringen Mengen gebildet wird und sich im Kreisgas ansammelt. Das entstandene Vinylacetat wird mit Hilfe geeigneter Methoden isoliert, die beispielsweise in der US 5,066,365 A beschrieben sind. Gleichwertige Verfahren sind auch für Allylacetat publiziert. Weiterhin ist bekannt, dass in derartigen Verfahren zur Herstellung von Vinylacetat auch gegebenenfalls im Katalysator enthaltene Promotoren, wie beispielsweise Kaliumacetat KOAc nachdosiert werden können, um während der Lebenszeit der Katalysatoren im Prozess verloren gegangene Anteile an Promotor wieder zuzuführen. Dies kann im laufenden Verfahren zur Herstellung von Alkenylcarbonsäureestern dadurch erfolgen, dass Azetatverbindungen der Promotoren in der dem Prozess zuzuführenden Essigsäure und/oder dem zuzuführenden Kaliumazetat beigemischt sind oder separat zudosiert werden.

Die Erfindung ist nachstehend anhand mehrerer Ausführungsbeispiele näher erklärt, ohne dass diese als einschränkend verstanden werden sollen.

In Fig. 1 sind exemplarisch für Katalysator 1 eine Auftragung der experimentellen Messpunkte der Pd-Konzentrationen und der mittels der gleichen Messung bestimmten Sigmoid-Funktion zur Bestimmung des Beginns der geometrischen Oberfläche nach Bestimmung von $x_{Oberfläche}$ dargestellt.

In Fig. 2 sind eine Auftragung der experimentellen Messpunkte der Pd-Konzentrationen und der mittels der entsprechenden Fit-Funktion erhaltene Kurvenverlauf von Katalysator 1 dargestellt.

In Fig. 3 sind eine Auftragung der experimentellen Messpunkte der Pd-Konzentrationen und der mittels der entsprechenden Fit-Funktion erhaltene Kurvenverlauf von Katalysator 2 dargestellt.

In Fig. 4 sind eine Auftragung der experimentellen Messpunkte der Pd-Konzentrationen und der mittels der entsprechenden Fit-Funktion erhaltene Kurvenverlauf von Katalysator 3 dargestellt.

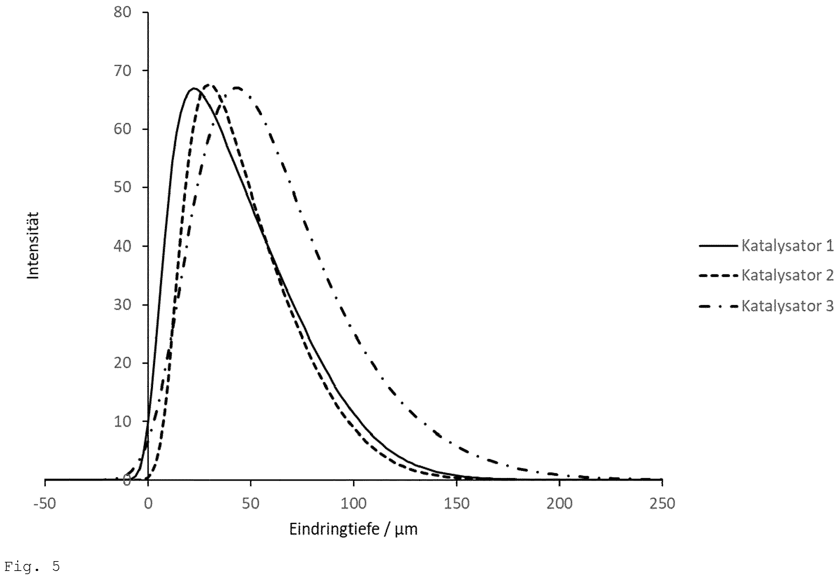

In Fig. 5 sind die mittels der entsprechenden Fit-Funktion erhaltenen Kurvenverläufe der Katalysatoren 1, 2 und 3 dargestellt.

Fig. 6 zeigt eine Darstellung der Selektivitäten bei unterschiedlichen Raumzeitausbeuten der Katalysatoren 1, 2 und deren zugehörige Trendlinien in der Reaktion von Essigsäure, Ethylen und Sauerstoff zu Vinylacetatmonomer.

Ausführungsbeispiele

Messmethoden

Porenvolumen

Das Porenvolumen wurde nach der Quecksilberporosimetrie-Methode gemäß DIN 66133 in einem Druckbereich von 1 bis 2000 bar gemessen.

BET-Oberfläche

Die BET-Oberflächen wurden nach DIN 66135 bestimmt. Zur Bestimmung des Mikroporenvolumens und der Mikroporen-Oberfläche wurde die Adsorptions-Isotherme von Stickstoff bei der Temperatur von flüssigem Stickstoff (77 K) mit dem ASAP 2020 M von Micromeritics bestimmt.

Elementverteilung im Trägermaterial

Die Verteilung des Pd im Katalysatorformkörper wurde ermittelt, indem ein Schnitt des kugelförmigen Katalysatorformkörpers hergestellt wurde, indem der Träger halbiert wird. Im Elektronenmikroskop ließ sich dann die räumliche Verteilung des Metalls mit Hilfe der EDX-Spektroskopie (energiedispersive Röntgenbeugung) ermitteln, die auch als EDX-Spektroskopie (Energy Dispersive X-ray) bezeichnet wird. Dabei wurde ein Messkopf über die Probe hinweg geführt, der sensitiv auf Pd reagiert, sodass dessen Verteilung entlang einer Linie von der äußeren Oberfläche hin zur Mitte des Katalysatorformkörpers ermittelt werden konnte.

Die Messung erfolgte an einem Elektronenmikroskop LEO 1530 der Firma LEO mit einer gekoppelten EDX-Einheit Quantax mit einem XFlash 4010 Detektor der Firma Bruker. Die Messbedingungen waren wie folgt:

Beschleunigungsspannung (EHT): 20 kV
Blende: 120 $\mu$m
Arbeitsabstand: ca. 16 mm (Optimum für REM-EDX Geometrie)
REM Detektor: SE2
Vergrößerung: niedrig (z. B. 55x)
Messdauer: ca. 10 - 15 min

Der Startpunkt der Messung wurde dabei mindestens 80 $\mu$m von der äußeren Kante der Probe gesetzt, und die Messung von dort über die Probe in Richtung Kugelmitte durchgeführt. Die Länge der Messlinie betrug 1600 $\mu$m.

Allgemein erfolgt die Messung für verschiedene Katalysatorgeometrien so, dass der Startpunkt der Messung mindestens 80 $\mu$m von der ursprünglichen geometrischen Oberfläche der Probe gesetzt wird, der Probenkopf von dort ausgehend senkrecht in Richtung der ursprünglichen geometrischen Oberfläche der Probe bewegt und darüber hinaus weiter über die Probe geführt wird.

Auf diese Weise ließ sich die Schalendicke des Pd bestimmen. Das innere Ende der Schalendicke wurde dabei als der Punkt definiert, an dem die Intensität erstmals einen Wert kleiner als die Summe aus Bremsstrahlung und deren dreifacher Standardabweichung aufweist.

Der Beginn der geometrischen Oberfläche des gemessenen Katalysatorformkörpers wurde dabei bestimmt, indem die Intensitäten der Bremsstrahlung innerhalb und außerhalb des Katalysatorformkörpers entlang der gemessenen Linie an verschiedenen Punkten x mit der Funktion

$$I(x) = base + \frac{max}{1 + e^{\frac{x_0 - x}{rate}}}$$

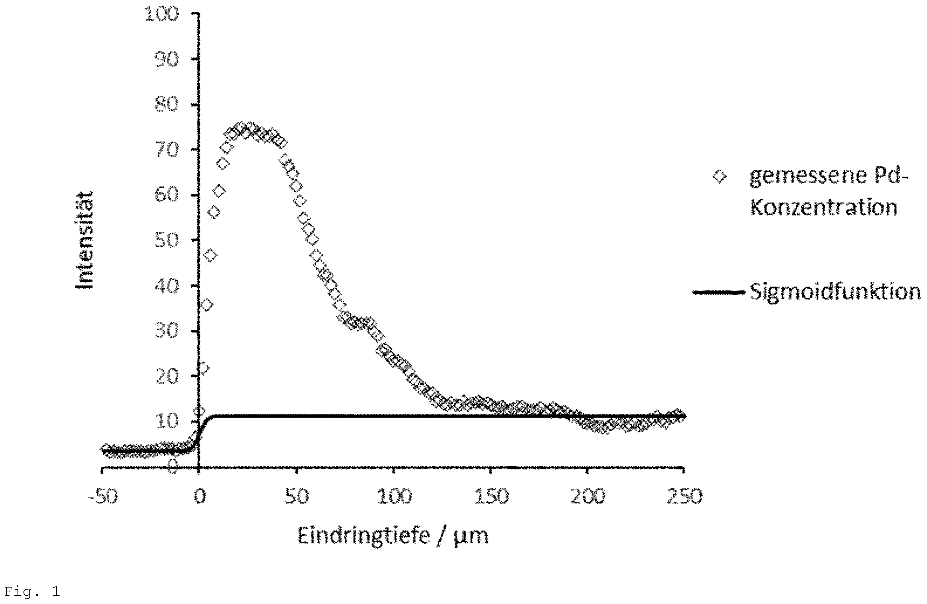

gefittet wurden. Dabei ist rate ein Maß für die Steilheit am Wendepunkt, base entspricht der Intensität I der Bremsstrahlung außerhalb des Katalysatorformkörpers und max entspricht der Intensität I der Bremsstrahlung innerhalb des Katalysatorformkörpers für den Bereich ohne Edelmetallbeladung, also außerhalb des Edelmetallschalenbereichs. Der Wendepunkt xo dieser Sigmoid-Funktion definiert hierbei das äußere Ende der Schale, und für die folgende Bestimmung der Konzentrationsverteilung der Edelmetalle entlang der gemessenen Linie wurde dieser Wert xo von den gemessenen x-Werten abgezogen, so dass alle Messreihen mit dem Punkt $x_{Oberfläche}$ = 0 $\mu$m der äußeren Oberfläche beginnen. In Fig. 1 ist exemplarisch anhand von Katalysator 1 eine graphische Darstellung der gemessenen Intensitäten und der mittels der gleichen Messung bestimmten Sigmoid-Funktion zur Bestimmung des Beginns der geometrischen Oberfläche nach Bestimmung von $x_{Oberfläche}$ dargestellt.

Auf diese Weise wurde eine ausreichende Anzahl an einzelnen Katalysatorformkörpern gemessen und deren äußere Oberfläche bestimmt.

Die Konzentrationsverteilung des Palladiums Edelmetalle entlang der Linie wurde bestimmt, indem von den einzelnen Messungen der arithmetische Mittelwert der Intensitäten am Punkt x berechnet wurde, und von den daraus resultierenden Messwerten mittels folgender Gauß-Funktion die Konzentrationsverteilung der Edelmetalle angefittet wurde:

$$I(x) = A \times e^{-\frac{x - x_{max}}{max} \times (1 + e^{\frac{x_{max} - x}{rate}})}$$

$x_{max}$ = Position des Peak-Maximums
A = bestimmt Peak-Höhe
max = bestimmt Asymmetrie der Konzentrationsverteilung
rate = bestimmt Asymmetrie der Konzentrationsverteilung

Auf diesem Wege wurden auch die Anteile der Halbwertsbreiten der Konzentrationsverteilung des Pd bestimmt. Die Halbwertsbreite ist hierbei definiert als Differenz zwischen den beiden Punkten entlang der Messlinie mit der halben Maximalintensität. Der linke Anteil der Halbwertsbreite ist dementsprechend definiert als Differenz zwischen dem ersten Punkt entlang der Messlinie mit halber Maximalintensität und dem Punkt mit Maximalintensität, während der rechte Anteil der Halbwertsbreite als die Differenz zwischen dem Punkt mit Maximalintensität und dem zweiten Punkt entlang der Messlinie mit halber Maximalintensität definiert ist.

Bestimmung der Elementkonzentrationen

Die Konzentration der Elemente erfolgte mittels dokimastischem Aufschluss über Kupferkuppellation und anschließender ICP-AES-Analyse und wurde berechnet in Bezug auf den Katalysatorformkörper nach Trocknung für 2 h bei 120 °C.

Bestimmung der Härte

Die Ermittlung der Härte der Katalysatorformkörper wurde mittels eines Tablettenhärtetesters 8M der Fa. Dr. Schleuniger Pharmatron AG an 99 Stück Katalysatorformkörpern als Durchschnitt bestimmt. Die Katalysatorformkörper wurden vor der Messung bei 130 °C für 2 h getrocknet. Die Geräteeinstellungen waren die folgenden:

| | |
|---|---|
| Härte: | N |
| Distanz zum Katalysatorformkörper: | 5,00 mm |
| Zeitverzögerung: | 0,80 s |
| Vorschub-Typ: | 6 D |
| Geschwindigkeit: | 0,60 mm/s |

Die nachstehenden Ausführungsbeispiele dienen der Erläuterung der Erfindung.

Beispiel 1: Katalysator 1

100 g des bentonithaltigen Katalysatorträgermaterials KA-160 (erhältlich bei Clariant) wurden eingewogen und gemäß der Porenfüllmethode (incipient-wetness) mit einer Mischung aus 39,3 g 2 molarer KOAc-Lösung und 18,1 g entionisiertem Wasser imprägniert. Nach statischer Trocknung im Wirbelschichttrockner bei 90°C für 35 min wurde die Mischung in einem Coater der Firma Innojet vom Typ IAC 025 mittels eines Prozessgases in eine Fließbettbewegung versetzt. Anschließend wurden 36,8 g entionisiertes Wasser in den Coater geleitet und auf den Katalysatorträger gesprüht. Die Prozessluft wurde auf eine Temperatur von 70 °C geregelt. Danach wurden 7,0 g einer wässrigen Cäsiumaurat-Lösung (4,7 Gew.-% Au) mit entionisiertem Wasser auf 50 g Coatinglösung verdünnt und diese in dem Coater bei einer Sprührate von 4 g/min und einer Temperatur von 70°C auf die Katalysatorträger in einem ersten Coatingschritt aufgebracht. Anschließend wurde in einem zweiten Coatingschritt eine Mischung von 2,7 g einer wässrigen Cäsiumaurat-Lösung (4,7 Gew.-% Au) und 38,9 g einer Tetramminpalladiumdihydroxid-Lösung (3,4 Gew.-% Pd) mit entionisiertem Wasser auf 80 g Coatinglösung verdünnt und mit einer Sprührate von 4 g/min und einer Temperatur von 70°C auf die Katalysatorträger aufgebracht. Dabei wurden die Katalysatorträger weiterhin in einem Fließbett gehalten. Nach erneuter statischer Trocknung im Wirbelschichttrockner (90°C/35 min) wurde der Katalysatorformkörper für 45 min bei 100°C mit Formiergas (2% $H_2$ in $N_2$) im Rohrofen statisch reduziert.

Die Elementaranalyse des Katalysatorformkörpers zeigte folgende Anteile:

| | |
|---|---|
| Pd: | 1,3 Gew.-% |
| Au: | 0,46 Gew.-% |

Die Bestimmung der Edelmetallverteilung erfolgte an einem Rasterelektronenmikroskop LEO 1530, ausgerüstet mit einem energiedispersiven Spektrometer der Fa. Bruker AXS. Zur Messung der Edelmetallkonzentration über die Schalendicke hinweg wurde ein Katalysatorformkörper durchschnitten, auf einen Aluminiumprobenhalter geklebt und anschließend mit Kohlenstoff bedampft. Als Detektor kam ein stickstofffreier Siliziumdriftkammerdetektor (XFlash® 410) mit einer Energieauflösung von 125 eV für die Mangan K-alpha-Linie zum Einsatz.

Die folgenden Parameter wurden für die Messung verwendet:

| | |
|---|---|
| Scanauflösung: | 500 Punkte |
| Abstand der Messpunkte: | 1,8 $\mu$m |
| Vergrößerung: | 200 fach |
| Strahlspannung | 20 kV |
| Strahlstrom | 20 nA |
| Eingangsimpulsrate: | 50000 Impulse/s |
| Messzeit für Linescan: | 200 s |

Von 10 Kugeln der wie vorstehend beschrieben hergestellten Schalenkatalysatorcharge wurde die Schalendicke vermessen.

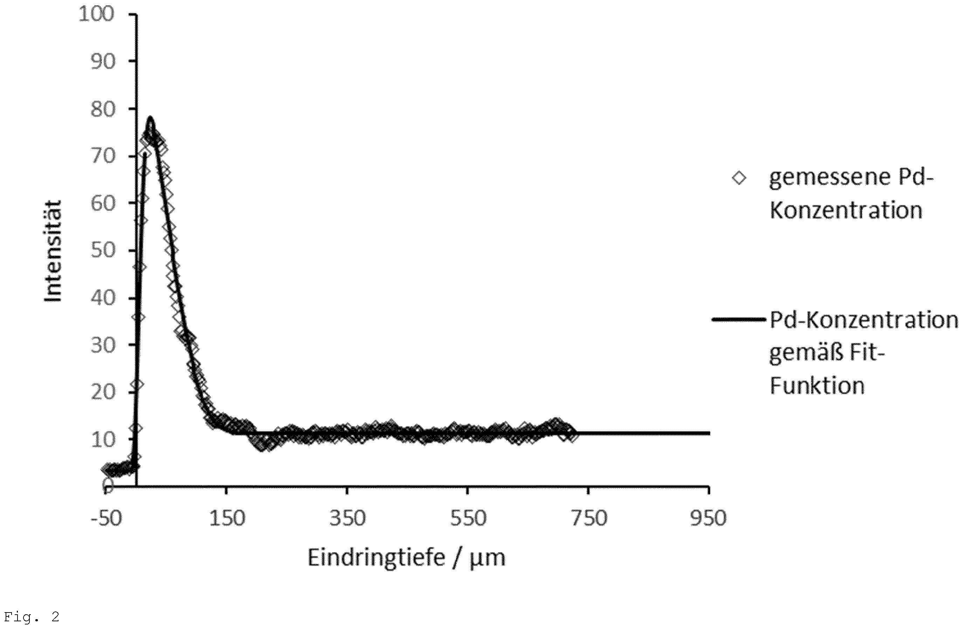

Das Maximum der Pd-Konzentration lag 23 Mikrometer unterhalb der geometrischen Oberfläche des Schalenkatalysators. Die Schalendicke für Pd betrug 130 Mikrometer. Die Halbwertsbreite für Pd betrug 60 Mikrometer, wobei der linke Anteil der Halbwertsbreite 16 Mikrometer und der rechte Anteil der Halbwertsbreite 44 Mikrometer betrug. Das Verhältnis linker zu rechter Anteil der Halbwertsbreite betrug somit 0.37. Der Kurvenverlauf der Pd-Konzentration ist in Fig. 2 dargestellt.

Beispiel 2:: Katalysator 2

100 g des bentonithaltigen Katalysatorträgermaterials KA-160 (erhältlich bei Clariant) wurden eingewogen und gemäß der Porenfüllmethode (incipient-wetness) mit einer Mischung aus 39,3 g 2 molarer KOAc-Lösung und 18,1 g entionisiertem Wasser imprägniert. Nach statischer Trocknung im Wirbelschichttrockner bei 90°C für 35 min wurde die Mischung in einem Coater der Firma Innojet vom Typ IAC 025 mittels eines Prozessgases in eine Fließbettbewegung versetzt. Die Prozessluft wurde auf eine Temperatur von 90 °C geregelt. Danach wurden 7,0 g einer wässrigen Cäsiumaurat-Lösung (4,7 Gew.-% Au) mit entionisiertem Wasser auf 50 g Coatinglösung verdünnt und diese in dem Coater bei einer Sprührate von 4 g/min und einer Temperatur von 70°C auf die Katalysatorträger in einem ersten Coatingschritt aufgebracht. Anschließend wurde in einem zweiten Coatingschritt eine Mischung von 2,4 g einer wässrigen Cäsiumaurat-Lösung (4,7 Gew.-% Au) und 38,9 g einer Tetramminpalladiumdihydroxid-Lösung (3,4 Gew.-% Pd) mit entionisiertem Wasser auf 80 g Coatinglösung verdünnt und mit einer Sprührate von 4 g/min und einer Temperatur von 70°C auf die Katalysatorträger aufgebracht. Dabei wurden die Katalysatorträger weiterhin in einem Fließbett gehalten. Nach erneuter statischer Trocknung im Wirbelschichttrockner (90°C/35 min) wurde der Katalysatorformkörper für 45 min bei 100°C mit Formiergas (2% $H_2$ in $N_2$) im Rohrofen statisch reduziert.

Die Elementaranalyse des Katalysatorformkörpers zeigte folgende Anteile:

| | |
|---|---|
| Pd: | 1,3 Gew.-% |
| Au: | 0,43 Gew.-% |

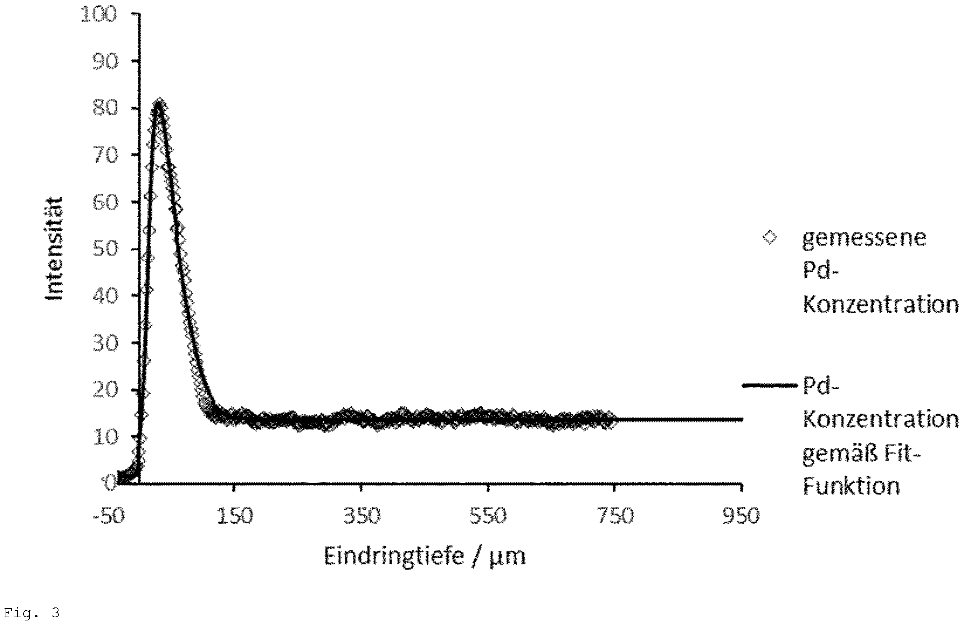

Die Bestimmung der Edelmetallverteilung erfolgte wie in Beispiel 1. Das Maximum der Pd-Konzentration lag 30 Mikrometer unterhalb der geometrischen, makroskopischen Oberfläche des Schalenkatalysators. Die Schalendicke für Pd betrug 129 Mikrometer. Die Halbwertsbreite für Pd betrug 50 Mikrometer, wobei der linke Anteil der Halbwertsbreite 16 Mikrometer und der rechte Anteil der Halbwertsbreite 34 Mikrometer betrug. Das Verhältnis linker zu rechter Anteil der Halbwertsbreite betrug somit 0.45. Der Kurvenverlauf der Pd-Konzentration ist in Fig. 3 dargestellt.

Vergleichsbeispiel 1: Katalysator 3

100 g des bentonithaltigen Trägermaterials KA-160 (erhältlich bei Clariant) wurden eingewogen und gemäß der Porenfüllmethode (incipient-wetness) mit einer Mischung aus 39,3 g 2 molarer KOAc-Lösung und 18,1 g entionisiertem Wasser imprägniert. Nach statischer Trocknung im Wirbelschichttrockner bei 90°C für 35 min wurde die Mischung in einem Coater der Firma Innojet vom Typ IAC 025 mittels eines Prozessgases in eine Fließbettbewegung versetzt und auf 70°C aufgeheizt. Anschließend wurden 7,0 g einer wässrigen Cäsiumaurat-Lösung (4,7 Gew.-% Au) mit entionisiertem Wasser auf 50 g Coatinglösung verdünnt und diese in dem Coater bei einer Sprührate von 4 g/min und einer Temperatur von 71°C auf die Trägerkörper in einem ersten Coatingschritt aufgebracht. Anschließend wurde in einem zweiten Coatingschritt eine Mischung von 2,4 g einer wässrigen Cäsiumaurat-Lösung (4,7 Gew.-% Au) und 38,9 g einer Tetramminpalladiumdihydroxid-Lösung (3,4 Gew.-% Pd) mit entionisiertem Wasser auf 80 g Coatinglösung verdünnt und mit einer Sprührate von 4 g/min und einer Temperatur von 70°C auf die Katalysatorträger aufgebracht. Dabei wurden die Katalysatorträger weiterhin in einem Fließbett gehalten. Nach erneuter statischer Trocknung im Wirbelschichttrockner (90°C/35 min) wurde der Katalysator für 45 min bei 100°C mit Formiergas (2% H2 in N2) im Rohrofen statisch reduziert.

Die Elementaranalyse des Katalysators zeigte folgende Anteile:

| | |
|---|---|
| Pd: | 1,3 Gew.-% |
| Au: | 0,43 Gew.-% |

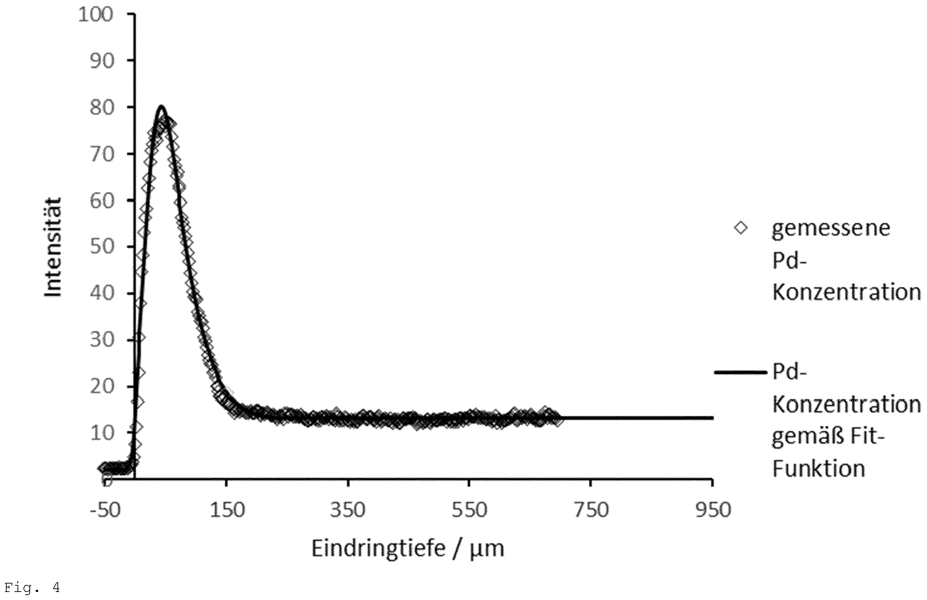

Die Bestimmung der Edelmetallverteilung erfolgte wie in Beispiel 1. Das Maximum der Pd-Konzentration lag 43 Mikrometer unterhalb der Oberfläche des Schalenkatalysators. Die Schalendicke für Pd betrug 183 Mikrometer. Die Halbwertsbreite für Pd betrug 73 Mikrometer wobei der linke Anteil der Halbwertsbreite 27 Mikrometer und der rechte Anteil der Halbwertsbreite 46 Mikrometer betrug. Das Verhältnis linker zu rechter Anteil der Halbwertsbreite betrug somit 0.60. Der Kurvenverlauf der Pd-Konzentration ist in Fig. 4 dargestellt.

Beispiel 3: Reaktortest

Testergebnisse der Katalysatoren 1, 2 und 3 hinsichtlich ihrer Aktivität, Selektivität und Produktivität bei der Synthese von Vinylacetatmonomer:

Für die katalytischen Tests wurden jeweils 2,9 g Katalysator in einen Reaktor mit einem Volumen von 5,7 ml gefüllt und anschließend unter einem Inertgas auf 138 °C erhitzt. Bei dieser Temperatur wurde der Inertgasstrom gegen einen Strom aus Essigsäure, Ethylen und Sauerstoff ersetzt, der durch den Reaktor geleitet wurde. Hinter dem Reaktor wurden in regelmäßigen Abständen Proben der Ausgangsströme entnommen und mittels Gaschromatographie analysiert. Nach einer Reaktionszeit von 24 Stunden bei 138 °C wurde die Reaktortemperatur auf 140 °C erhöht und für weitere 12 Stunden belassen. Anschließend erfolgten weitere Temperaturerhöhungen auf 142° C, 144° C, 146° C und abschließend wieder auf 140° C. Die jeweiligen Reaktionszeiten betrugen jeweils 12 Stunden, der Druck betrug 5-6 barg. Die Konzentrationen der verwendeten Komponenten betrugen: 45% Ethylen, 6% $O_2$, 0,9% $CO_2$, 9% Methan, 15,5% Essigsäure, Rest $N_2$.

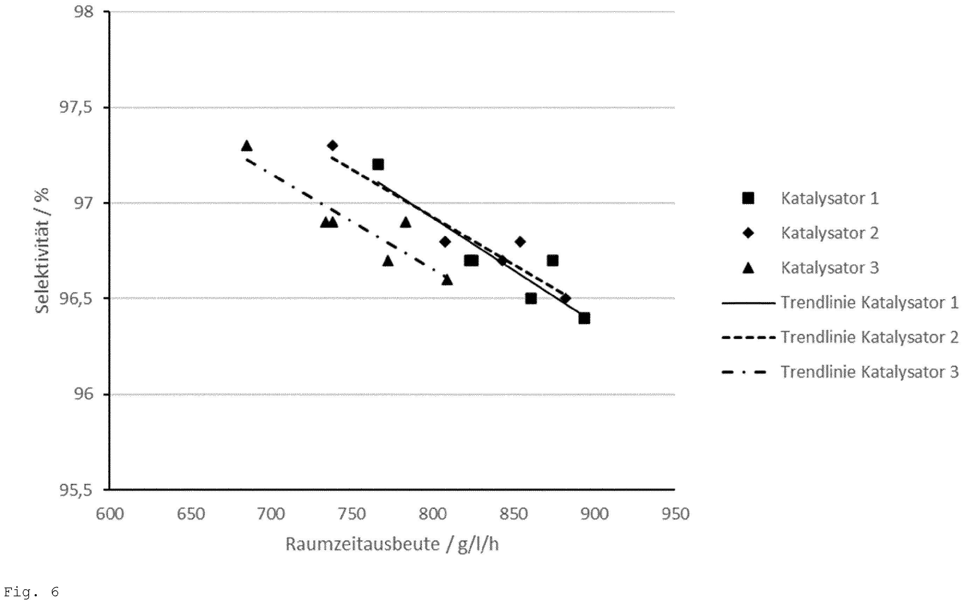

Tabelle 1 und Figur 6 zeigen die Selektivität bzw. die Aktivität der Katalysatoren 1, 2 und 3 in Abhängigkeit von der $O_2$-Umwandlung. Daraus geht eindeutig hervor, dass die erfindungsgemäß hergestellten Katalysatoren 1 und 2 eine weitaus höhere Aktivität, Selektivität (bei gleichem Aktivitätslevel) und Produktivität aufweisen, als der Vergleichskatalysator, Katalysator 3. Dies wird zusätzlich in Fig. 6 veranschaulicht, in der eine Auftragung der Selektivität zu VAM in Abhängigkeit der Raumzeitausbeute dargestellt ist.

Tabelle 1: katalytische Testdaten der Katalysatoren 1 bis 3

| Beispiel | Temperatur [°C] | 138 | 140 | 142 | 144 | 146 | 140 |
|---|---|---|---|---|---|---|---|
| | Druck [barg] | 6 | 6 | 5 | 5 | 5 | 5 |
| Katalysator 1 | Ausbeute [g/(L·h)] | 823 | 874 | 825 | 861 | 894 | 766 |
| | Selektivität [%] | 96,7 | 96,7 | 96,7 | 96,5 | 96,4 | 97,2 |
| | $O_2$-Umsatz [%] | 73,4 | 76,9 | 72,4 | 75,8 | 79,7 | 65,1 |
| Katalysator 2 | Ausbeute [g/(L·h)] | 808 | 854 | 808 | 843 | 882 | 738 |
| | Selektivität [%] | 96,8 | 96,8 | 96,8 | 96,7 | 96,5 | 97,3 |
| | $O_2$-Umsatz [%] | 71,2 | 74,6 | 70,4 | 73,9 | 77,2 | 62,1 |
| Katalysator 3 | Ausbeute [g/(L·h)] | 734 | 783 | 738 | 772 | 809 | 685 |
| | Selektivität [%] | 96,9 | 96,9 | 96,9 | 96,7 | 96,6 | 97,3 |
| | $O_2$-Umsatz [%] | 64,8 | 68,7 | 64,4 | 68,3 | 71,1 | 57,8 |

**Patentansprüche**

1. Pd- und Au-haltiger Schalenkatalysator, **dadurch gekennzeichnet, dass** er einen Konzentrationsverlauf des Palladiums innerhalb des Schalenkatalysators aufweist, bei dem der Konzentrationsverlauf einer asymmetrischen Gauß-Verteilung entspricht, sich das Maximum der Palladiumkonzentration in einem Bereich von 0 bis 40 Mikrometer ausgehend von der geometrischen, makroskopischen Oberfläche des Katalysatorformkörpers befindet, das Palladium in einem Bereich mit einer Schalendicke von 30 bis 200 $\mu$m vorliegt, die Halbwertsbreite der Konzentrationsverteilung 10 bis 70 $\mu$m beträgt und das Verhältnis linkem Anteil zu rechtem Anteil der Halbwertsbreite im Bereich von 0,05 bis 0,55 liegt.

2. Schalenkatalysator nach Anspruch 1, wobei das Maximum der Pd-Konzentration im Bereich von 5 bis 40 Mikrometer, bevorzugt 5 bis 35 Mikrometer, besonders bevorzugt 5 bis 30 Mikrometer, ausgehend von der Oberfläche des Schalenkatalysators, liegt.

3. Schalenkatalysator nach einem der vorherigen Ansprüche, wobei die Schale umfassend Pd eine Dicke von 30 bis 200 Mikrometer, bevorzugt 40 bis 180 Mikrometer, besonders bevorzugt 50 bis 160 Mikrometer, am bevorzugtesten 60 bis 140 Mikrometer, aufweist.

4. Schalenkatalysator nach einem der vorherigen Ansprüche, weiterhin umfassend ein Alkalimetallacetat, bevorzugt Kaliumacetat.

5. Schalenkatalysator nach einem der vorherigen Ansprüche, wobei der Anteil an Pd im Bereich von 0,2 bis 2,0 Gew.-%, bevorzugt im Bereich von 0,4 bis 1,75 Gew.-% und besonders bevorzugt im Bereich von 0,7 bis 1,5 Gew.-%, liegt, und der Anteil an Au im Bereich von 0,1 bis 1,2 Gew.-%, bevorzugt im Bereich von 0,2 bis 1,0 Gew.-% und am bevorzugtesten im Bereich von 0,3 bis 0,8 Gew.-%, liegt, jeweils bezogen auf das Gesamtgewicht des Katalysatorformkörpers nach Reduktion und Trockenverlust.

6. Verfahren zur Herstellung eines Pd- und Au-haltigen Schalenkatalysators nach einem der Ansprüche 1 bis 5, umfassend die folgenden Schritte:

    (a) Unterziehen einer Schüttung eines Katalysatorträgers einer Umwälzbewegung, wobei die Schüttung vor oder nach Unterziehen der Umwälzbewegung auf eine Temperatur von 60 bis 120 erwärmt wird;

    Sequentielles oder gleichzeitiges Aufbringen einer gelösten Pd-haltigen Vorläuferverbindung und einer gelösten Au-haltigen Vorläuferverbindung durch Aufsprühen auf die der Umwälzbewegung unterzogenen Schüttung des Katalysatorträgers, wobei bei einer gleichzeitigen Aufbringung die Temperatur 5 bis 30 °C, bevorzugt 7 bis 25 °C, am bevorzugtesten 10 bis 20 °C unter der in Schritt (a) eingestellten Temperatur liegt und wobei bei einer sequentiellen Aufbringung die Temperatur der ersten Aufbringung 5 bis 30 °C, bevorzugt 7 bis 25 °C, am bevorzugtesten 10 bis 20 °C unter der in Schritt (a) eingestellten Temperatur liegt.

7. Verfahren nach Anspruch 6, wobei nach Schritt (b) in einem Schritt (c) eine Reduktion der Metallkomponenten der Vorläuferverbindungen zu den elementaren Metallen durch Unterziehen des in Schritt (b) erhaltenen Katalysatorträgerkörpers mittels einer Temperaturbehandlung in einer nicht-oxidierenden Atmosphäre stattfindet.

8. Verfahren nach Anspruch 6 oder 7, wobei die Temperatur der Aufbringung, bzw. im Fall einer sequentiellen Aufbringung die Temperatur der ersten Aufbringung in Schritt (b) im Bereich von 55 bis 115 °C, bevorzugt im Bereich von 55 bis 110 °C, mehr bevorzugt im Bereich von 60 bis 100 °C, besonders bevorzugt im Bereich von 65 bis 90 °C liegt.

9. Verfahren nach einem der Ansprüche 6 bis 8, wobei in Schritt (b) in einem ersten Schritt eine gelöste Au-haltige Vorläuferverbindung und in einem zweiten Schritt eine gelöste Pd-haltige Vorläuferverbindung aufgebracht wird.

10. Verfahren nach einem der Ansprüche 6 bis 9, wobei vor der Aufbringung der Pd- und Au-haltigen Vorläuferverbindungen ein Acetat auf die Katalysatorträger aufgebracht wird, bevorzugt vor Schritt (a).

11. Verfahren zur Herstellung eines Pd- und Au-haltigen Schalenkatalysators nach einem der Ansprüche 1 bis 5, umfassend die folgenden Schritte:

    (a) Unterziehen einer Schüttung eines Katalysatorträgers einer Umwälzbewegung und In-Kontaktbringen mit der Schüttung mit zerstäubtem Wasser durch Aufsprühen bei einer Temperatur im Bereich von 55 bis 110°C;
    (b) Sequentielles oder gleichzeitiges Aufbringen einer gelösten Pd-haltigen Vorläuferverbindung und einer gelösten Au-haltigen Vorläuferverbindung durch Aufsprühen auf die der Umwälzbewegung unterzogenen Schüttung des Katalysatorträgers, wobei bei einer gleichzeitigen Aufbringung die Temperatur dieselbe wie in Schritt (a) ist, und wobei bei einer sequentiellen Aufbringung die Temperatur der ersten Aufbringung dieselbe wie in Schritt (a) ist.

12. Verfahren nach Anspruch 11, wobei nach Schritt (b) in einem Schritt (c) eine Reduktion der Metallkomponenten der Vorläuferverbindungen zu den elementaren Metallen durch Unterziehen des in Schritt (b) erhaltenen Katalysatorträgerkörpers mittels einer Temperaturbehandlung in einer nicht-oxidierenden Atmosphäre stattfindet.

13. Verfahren nach Anspruch 11 oder 12, wobei in Schritt (b) in einem ersten Schritt eine gelöste Au-haltige Vorläuferverbindung und in einem zweiten Schritt eine gelöste Pd-haltige Vorläuferverbindung aufgebracht wird.

14. Verfahren nach einem der Ansprüche 11 bis 13, wobei vor der Aufbringung der Pd- und Au-haltigen Vorläuferverbindungen ein Acetat auf die Katalysatorträger aufgebracht wird, bevorzugt vor Schritt (a).

15. Verfahren zur Herstellung von Alkenylcarbonsäureestern, insbesondere VAM oder Allylacetatmonomer mit dem Schalenkatalysator gemäß einem der Ansprüche 1 bis 5.

gemessene Pd-Konzentration

Sigmoidfunktion

Intensität

Eindringtiefe / µm

Fig. 1

Fig. 2

Fig. 3

Fig. 4

Fig. 5

Fig. 6

Europäisches
Patentamt

European
Patent Office

Office européen
des brevets

# EUROPÄISCHER RECHERCHENBERICHT

**Nummer der Anmeldung**

**EP 21 21 6649**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2010/217052 A1 (UNGAR SYBILLE [DE] ET AL) 26. August 2010 (2010-08-26) * Absätze [0023] – [0027], [0035], [0036], [0045] – [0047]; Abbildungen 1-3 * | 1-15 | INV. B01J35/00 B01J23/44 B01J23/52 C07C67/055 |
| | ----- | | |
| A | US 2010/273644 A1 (HAGEMEYER ALFRED [DE] ET AL) 28. Oktober 2010 (2010-10-28) * Absätze [0104] – [0111] * | 1-15 | |
| | ----- | | |
| A | DE 10 2012 008714 A1 (CLARIANT PRODUKTE DEUTSCHLAND [DE]) 7. November 2013 (2013-11-07) * Absätze [0045] – [0050] * | 1-15 | |
| | ----- | | |
| A | DE 10 2012 008715 A1 (CLARIANT PRODUKTE DEUTSCHLAND [DE]) 7. November 2013 (2013-11-07) * Absätze [0022], [0023], [0046] – [0051] * | 1-15 | |
| | ----- | | RECHERCHIERTE SACHGEBIETE (IPC) |
| A | DE 10 2010 026462 A1 (SUED CHEMIE AG [DE]) 12. Januar 2012 (2012-01-12) * Absätze [0066] – [0068], [0086] – [0093]; Abbildungen 1-8 * | 1-15 | B01J C07C |
| | ----- | | |

Der vorliegende Recherchenbericht wurde für alle Patentansprüche erstellt

| Recherchenort | Abschlußdatum der Recherche | Prüfer |
|---|---|---|
| Den Haag | 23. Mai 2022 | Viskanic, Martino |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 21 21 6649

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-05-2022

| Im Recherchenbericht angeführtes Patentdokument | Datum der Veröffentlichung | Mitglied(er) der Patentfamilie | Datum der Veröffentlichung |
|---|---|---|---|
| US 2010217052 A1 | 26-08-2010 | CN 101730588 A | 09-06-2010 |
|  |  | DE 102007025315 A1 | 11-12-2008 |
|  |  | EP 2155392 A2 | 24-02-2010 |
|  |  | JP 5323060 B2 | 23-10-2013 |
|  |  | JP 2010527776 A | 19-08-2010 |
|  |  | KR 20100041714 A | 22-04-2010 |
|  |  | RU 2009145197 A | 10-07-2011 |
|  |  | TW 200916189 A | 16-04-2009 |
|  |  | US 2010217052 A1 | 26-08-2010 |
|  |  | WO 2008145387 A2 | 04-12-2008 |
| US 2010273644 A1 | 28-10-2010 | CN 102015103 A | 13-04-2011 |
|  |  | DE 102007025362 A1 | 11-12-2008 |
|  |  | JP 2010535607 A | 25-11-2010 |
|  |  | US 2010273644 A1 | 28-10-2010 |
|  |  | WO 2008145395 A2 | 04-12-2008 |
| DE 102012008714 A1 | 07-11-2013 | CN 104364012 A | 18-02-2015 |
|  |  | DE 102012008714 A1 | 07-11-2013 |
|  |  | EP 2844391 A1 | 11-03-2015 |
|  |  | JP 5684851 B2 | 18-03-2015 |
|  |  | JP 2014012268 A | 23-01-2014 |
|  |  | TW 201347846 A | 01-12-2013 |
|  |  | US 2015126360 A1 | 07-05-2015 |
|  |  | WO 2013164458 A1 | 07-11-2013 |
| DE 102012008715 A1 | 07-11-2013 | CN 104379259 A | 25-02-2015 |
|  |  | DE 102012008715 A1 | 07-11-2013 |
|  |  | EP 2844390 A1 | 11-03-2015 |
|  |  | JP 5684852 B2 | 18-03-2015 |
|  |  | JP 2014012269 A | 23-01-2014 |
|  |  | TW 201347847 A | 01-12-2013 |
|  |  | TW 201603884 A | 01-02-2016 |
|  |  | US 2015126361 A1 | 07-05-2015 |
|  |  | WO 2013164454 A1 | 07-11-2013 |
| DE 102010026462 A1 | 12-01-2012 | CN 103025423 A | 03-04-2013 |
|  |  | DE 102010026462 A1 | 12-01-2012 |
|  |  | DE 112011102295 A5 | 25-04-2013 |
|  |  | JP 5723002 B2 | 27-05-2015 |
|  |  | JP 2013534471 A | 05-09-2013 |
|  |  | US 2013172603 A1 | 04-07-2013 |
|  |  | WO 2012004334 A2 | 12-01-2012 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008145388 A1
- WO 2005065821 A1

- US 5066365 A

**In der Beschreibung aufgeführte Nicht-Patentliteratur**

- **HOLLEMANN WIBERG ; DE GRUYTER.** Lehrbuch der anorganischen Chemie. 2007
- Römpp Lexikon Chemie. Georg Thieme Verlag

- Römpp, Lexikon Chemie. Georg Thieme Verlag